(19) Europäisches Patentamt
European Patent Office
Office européen des brevets

(11) Veröffentlichungsnummer: **0 461 419 A1**

# EUROPÄISCHE PATENTANMELDUNG

(12)

(21) Anmeldenummer: **91108091.9**

(22) Anmeldetag: **18.05.91**

(51) Int. Cl.$^5$: **C07C 219/06**, C11D 1/46, A01N 37/12

(30) Priorität: **12.06.90 DE 4018750**

(43) Veröffentlichungstag der Anmeldung:
**18.12.91 Patentblatt 91/51**

(84) Benannte Vertragsstaaten:
**AT BE CH DE ES FR GB IT LI LU NL SE**

(71) Anmelder: **REWO Chemische Werke GmbH**
**Industriegebiet West**
**W-6497 Steinau an der Strasse(DE)**

(72) Erfinder: **Euler, Axel**
**Hollerain 3**
**W-6497 Steinau an der Strasse(DE)**
Erfinder: **Köhle, Hans-Jürgen, Dr. Dipl.-Chem.**
**Lotichiusstrasse 13**
**W-6490 Schlüchtern(DE)**
Erfinder: **Schiesser, Hans-Georg**
**Reichenaustrasse 6**
**W-6483 Bad Soden-Salmünster(DE)**
Erfinder: **Wehner, Winfried, Dr. Dipl.-Chem.**
**August Rosterg Strasse 32**
**W-6404 Neuhof(DE)**
Erfinder: **Weigand, Joachim, Dr. Dipl.-Chem.**
**Haupstrasse 71**
**W-6463 Freigericht 1(DE)**

(54) **Poly(oxyalkylen)aminoalkanolester, deren Ammoniumverbindungen, Verfahren zu ihrer Herstellung und ihre Verwendung in Emulgatoren, Reinigungsmitteln, Desinfektionsmitteln und Konservierungsmitteln.**

(57) Gegenstand der Erfindung sind Poly(oxyalkylen) aminoalkanolester, deren Ammoniumverbindungen, Verfahren zu ihrer Herstellung und ihre Verwendung in Emulgatoren, Reinigungsmitteln , Desinfektionsmitteln und Konservierungsmitteln auf Basis von Verbindungen der allgemeinen Formel

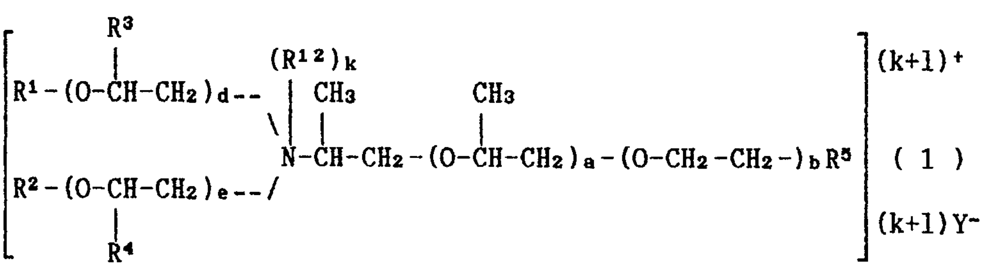

( 1 )

EP 0 461 419 A1

Gegenstand der Erfindung sind Poly(oxyalkylen)-aminoalkanolaminester der allgemeinen Formel (1):

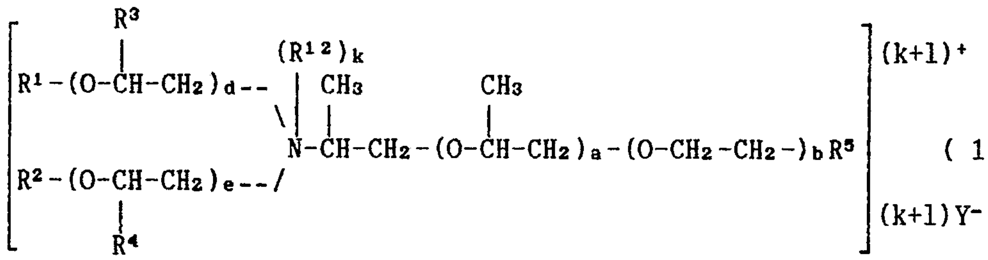

(1)

in welcher $R^5$ ein Rest

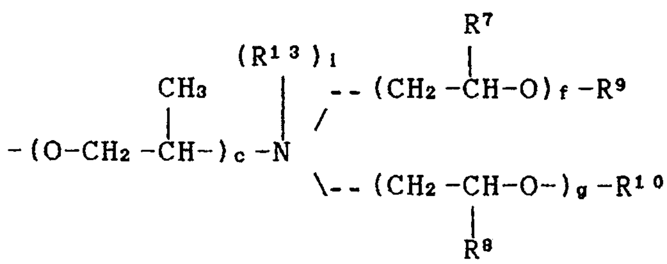

oder -OH oder -O-$R^6$ sein kann, mit $R^6$ = Alkylrest mit 1 - 5 C-Atomen oder ein Rest

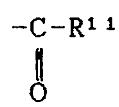

und worin $R^1$, $R^2$, $R^9$, $R^{10}$ unabhängig voneinander Wasserstoff, ein Alkylrest mit 1- 5 Kohlenstoffatomen vorzugsweise -$CH^3$ oder der Rest

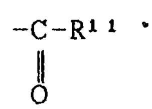

sein können, in welchem $R^{11}$ ein gegebenenfalls verzweigter, gegebenenfalls Mehrfachbindungen oder Hydroxylgruppen enthaltender Kohlenwasserstoffrest mit 6 - 21, vorzugsweise 7 - 17 Kohlenstoffatomen ist, mit der Bedingung, daß mindestens einer der Reste $R^1$, $R^2$, $R^9$, $R^{10}$ der Rest

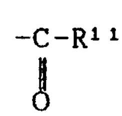

sein muß und $R^3$, $R^4$, $R^7$, $R^8$ unabhängig voneinander -H oder -$CH_3$ sein können und $R^{12}$, $R^{13}$ unabhängig voneinander -H, -$CH_3$, $C_2H_5$ oder ein Benzylrest sein können und k und 1 = 0 bis 1 und k + 1 = 0 bis 2 und a + c = 1 - 40, vorzugsweise 1 bis 15, b = 0 - 40, vorzugsweise 0 bis 15, d + e + f + g = 2 - 12 sind und $y^-$ für ein ein- oder mehrwertiges Anion steht, vorzugsweise Lactat, Methosulfat, Sulfat, Chlorid mit insgesamt k + 1 negativen Ladungen.

Weitere Gegenstände der Erfindung ist ein Verfahren zur Herstellung dieser Verbindungen, sowie ihre Verwendung für die Herstellung von Emulgatoren, Reinigungsmitteln, Desinfektionsmitteln.

Ausgangsverbindungen zur Herstellung der Esteramine können die folgenden Aminverbindungen der Formeln (2) und (3) eingesetzt werden:

$$H_2N-CH(CH_3)-CH_2-(PO)_a-(EO)_b-(PO)_c-CH_2-CH(CH_3)-NH_2 \qquad (2)$$

$$H_2N-CH(CH_3)-CH_2-(PO)_a-(EO)_b-(PO)_c-O-R^6 \qquad (3)$$

worin

$$PO \quad -(O-CH_2-CH(CH_3))$$

und EO $-(O-CH_2-CH_2)-$ bedeuten und worin a = 0 - 20, C = 0 - 20 und a , c , b und $R^6$ die oben angegebene Bedeutung haben, $R^6$ aber insbesondere ein Alkylrest mit 1 - 5 Kohlenstoffatomen und C = 0 ist.

Diese Verbindungen sind handelsüblich und werden nach bekannten Vefahren durch die Umsetzung von Polyoxyalkylenalkoholen mit Ammoniak unter Druck gewonnen.

Die Polyoxyalkylenalkohole werden hergestellt durch Addition eines Alkylenoxides, im wesentlichen Propylenoxid, Ethylenoxid oder eine Mischung aus beiden, unter Anwendung eines üblichen Verfahrens an eine Verbindung, die ein oder mehrere aktive Wasserstoffatome enthält oder durch Polymerisation von Alkylenoxiden.

Als Verbindungen, welche ein oder mehrere aktive Wasserstoffatome enthalten, können Monoalkohole wie Ethanol, Isopropanlol, Butanol, Laurylalkohol, Stearylalkohol, insbesondere aber Methanol oder Glykole wie Ethylenglykol, Propylenglykol, Diethylenglykol, Glycerin, Trimethylolpropan, Pentaerythrit, Sorbit, Polyglycerin, Polyvinylalkohole verwendet werden.

Die Polyoxyalkylenalkohole weisen Molgewichte im Bereich von ca. 100 bis 10.000, vorzugsweise ca. 150 - 5.000 und besonders bevorzugt ca. 150 - 2.000 auf.

Die weitere Umsetzung zu den Aminen erfolgt nach an sich bekannten Methoden durch Aminolyse der freien Hydroxylgruppen oder deren Ester, insbesondere der Schwefelsäureester. Bei höheren Alkoholen erfolgt der Austausch der OH-Gruppe gegen die Aminogruppe mittels homogener, insbesondere aber heterogener Katalyse an Festkontakten. Hier stehen insbesondere zwei Methoden zur Verfügung. Die eine arbeitet mit dehydratisierenden, die andere mit hydrierenddehydrierend wirkenden Katalysatoren.

Als Katalysatoren werden Aluminiumoxyd mit Zusätzen von bis zu ca. 20 % an Chrom-III-oxid, Zinn-II-oxid, Zinkoxyd, Eisen-III-oxid verwendet.

Aluminiumoxyd, auf Silicagel oder Aktivkohle niedergeschlagen, eignet sich unter anderem zur drucklosen Umwandlung höherer Fettalkohole in Fettamine (vergl. GB-PS 384 714, US-PS 2 017 051, US-PS 2 078 922). Häufig benutzt werden Aluminiumphosphatsäuren oder Phosphorsäuren allein oder auf Trägermaterialien wie Silicagel oder Kieselgur (US-PS 2 073 671) für die Herstellung bei normalem oder erhöhtem Druck (US-PS 2 043 965). Die Arbeitstemperaturen liegen je nach Katalysator im Bereich zwischen 200 - 600 °C. Über Temperatur- bzw. Druckeinfluß und Ammoniaküberschuß sowie die erforderlichen Verweilzeiten gibt es jeweils umfassende Literaturangaben (vgl. Houben-Weyl, Mehtoden der organischen Chemie, Georg Thieme Verlag, Stuttgart 1957, Band 11/1 S. 108ff.).

Erfindungsgemäß wurden folgende Verbindungen nach Formel (2) bevorzugt:

$$a + c = 2 - 6 \qquad (I)$$
$$b = 0$$

oder

$$a + c = 2 - 3 \quad \text{(II)}$$
$$b = 6 - 9$$

und nach Formel (3)

$$a + c = 6 - 10 \quad \text{(III)}$$
$$b = 1 - 2$$
$$R^6 = -CH_3$$

Die Verbindungen nach Formel (2) und (3) werden anschliessend nach an sich bekannten Verfahren alkoxiliert, d. h. vorzugsweise ethoxyliert bzw. propoxyliert. Im allgemeinen wird dabei so verfahren, daß man die Amine in einem Druckreaktor bei 120 - 160 °C, gegebenenfalls in Gegenwart basischer, insbesondere alkolischer Katalysatoren bei 1 - 4 bar mit einer dem gewünschten Alkoxilierungsgrad entsprechenden Menge an Alkylenoxid, erfindungsgemäß bevorzugt sind Ethylenoxid und Propylenoxid oder deren Mischungen, abreagiert.

Erhalten werden Verbindungen der allgemeinen Formel (4) und (5),

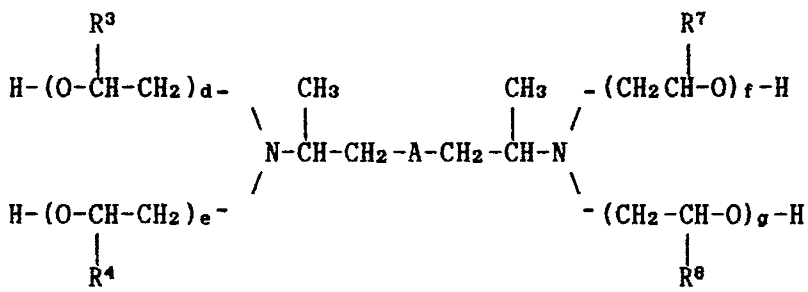

( 4 )

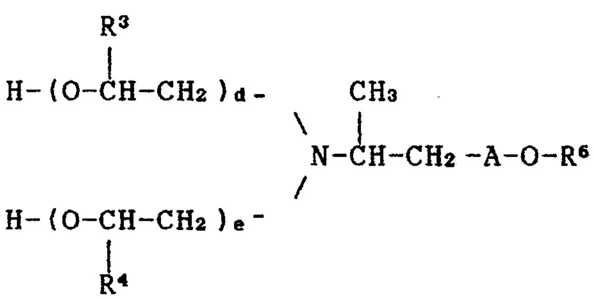

( 5 )

worin A $-(PO)_a-(EO)_b-(PO)_c$ bedeutet, und worin a, b, c, und $R^6$, EO und PO die gleiche Bedeutung wie oben aufgeführt haben und

$d + e + f + g = 2 - 12$ und
$R^3$, $R^4$ $R^7$, $R^8$ = H

unabhängig voneinander -H oder $-CH_3$ sein können.

Bevorzugte Verbindungen nach Formel (4) sind Verbindungen mit

$d + e + f + g = 4 - 6$ (IV)
$R^3$, $R^4$ $R^7$, $R^8$ = H

und nach Formel (5) mit

$d + e = 2 - 3$ (V)
$R_3, R_4 = H$

Die anschließende Veresterung der Verbindungen (4) bzw. (5) mit Carbonsäuren oder deren Derivaten führt zu Verbindungen der allgemeinen Formeln (6) und (7)

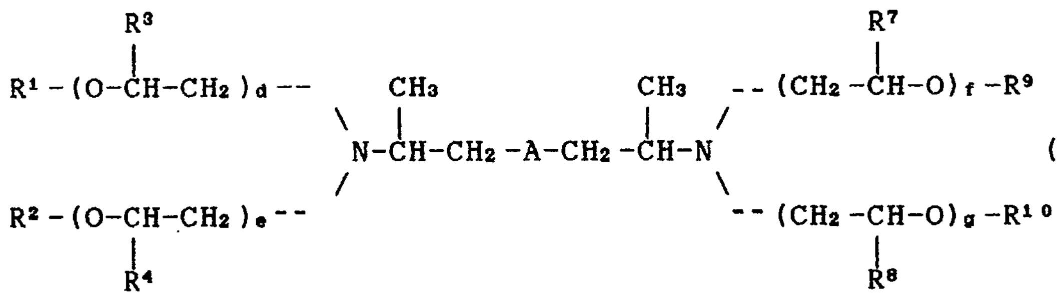

( 6 )

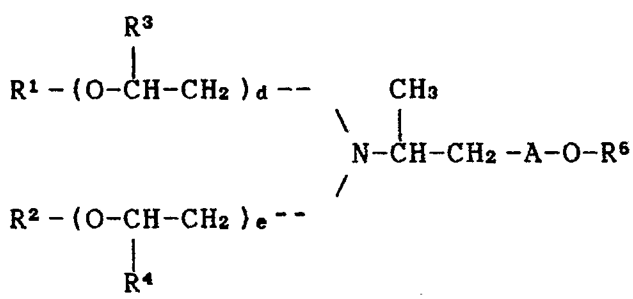

( 7 )

wobei a, b, c, d, e, f, g, $R^3$, $R^4$, $R^7$, $R^8$, $R^6$ und A die gleiche Bedeutung wie oben aufgeführt haben und $R^1$, $R^2$, $R^9$, $R^{10}$ unabhängig voneinander ein H, ein Alkylrest mit 1 - 5 kohlenstoffatomen oder ein

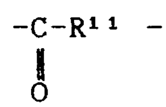

Rest ist, wobei gegebenenfalls verzweigter, gegebenenfalls Mehrfachbindung oder Hydroxylgruppen enthaltender Kohlenwasserstoffrest mit 6 - 21 C-Atomen ist, vorzugsweise 7 - 17 C-Atomen, mit der Bedingung, daß mindestens einer der Reste $R^1$, $R^2$, $R^9$, $R^{10}$ ein Rest

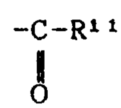

sein muß.

Als Fettsäuren für die Veresterung, bzw. die Umesterung werden die auf diesem Gebiet bekannten und üblichen einbasischen synthetischen Fettsäuren, insbesondere aber die Fettsäuren auf Basis natürlicher pflanzlicher und tierischer Öle mit 6 - 22 C-Atomen, insbesondere mit 8 - 18 C-Atomen eingesetzt, wie beispielsweise Kokosfettsäuren, Palm-, Talg-, Ricinusfettsäuren. Diese können sowohl als Glyceride, als Ester mit kurzkettigen Alkoholen oder als freie Säuren eingesetzt werden.

Ihre Veresterung oder Umesterung wird nach dem bekannten Verfahren durchgeführt.

Hierbei werden die Alkanolamine mit einer dem gewünschten Veresterungsgrad entsprechenden Menge an Fettsäure oder Fettsäureester, gegebenenfalls in Gegenwart eines Katalysators, bei 160 - 240 °C

umgesetzt und das sich bildende Reaktionswasser, bzw. der Alkohol, kontinuierlich abdestilliert, wobei zur Vervollständigung der Reaktion gegebenenfalls ein Unterdruck angelegt wird.

Bevorzugte Verbindungen nach Formel (6) sind Substanzen mit

$R^1$, $R^2$, $R^9$, $R^{10}$ = 2 x H

2 x 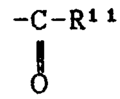 (VI)

wobei $R^{11}$ = $-C_{17}H_{35}$ bedeutet und nach Formel (7) mit

$R^1$ = H

$R^2$ = 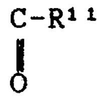 (VII)

wobei $R^{11}$ = $-C_{17}H_{35}$ ist.

Die Quaternierung bzw. die Herstellung der Salze der Verbindungen (6) und (7) wird nach den auf diesem Gebiet bekannten Verfahren durchgeführt und führt zu den erfindungsgemäßen Esteraminquats bzw. Esteraminsalzen der allgemeinen Formel (1), worin $R^{12}$ und $R^{13}$ die angegebene Bedeutung haben, k und 1 größer als Null sind und k + 1 vorzugsweise Werte zwischen 0,5 - 2 hat.

Die Herstellung der Salze erfolgt im allgemeinen so, daß man die Säuren gegebenenfalls als wässrige oder alkoholische Lösungen in einer dem gewünschten Salzbildungsgrad entsprechenden Menge bei 20 - 80 °C den vorgelegten Poly(oxyalkylen)alkanolaminestern und bei gutem Rühren gegebenenfalls unter Kühlung portionsweise zugibt. Die Quaternierung erfolgt entsprechnd den allgemein bekannten Verfahren, wobei die Poly(oxyalkylen)alkanol-aminester, gegebenenfalls unter Mitverwendung eines Lösungsmittels, auf 40 - 80 °C aufgeheizt und portionsweise mit dem Quaternierungsmittel in einer dem gewünschten Quaternierungsgrad entsprechenden Menge versetzt werden.

Als Anionen kommen demnach bevorzugt in Betracht:

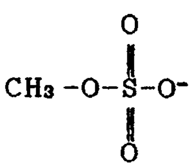 , 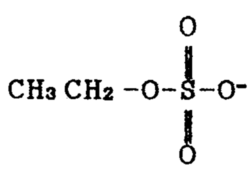 , $HCOO^-$ , $CH_3COO^-$

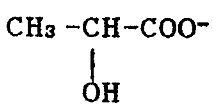 , $OHCH_2COO^-$ , 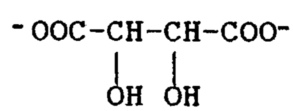 ,

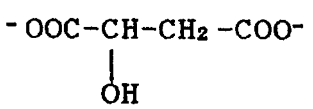 , 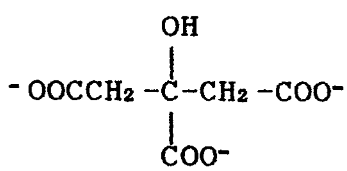 , $C_6H_5COO^-$ ,

$^-OOC-(CH_2)_n-COO^-$ , $Cl^-$ , $Br^-$ , $J^-$ , $SO_4^{2-}$ , $PO_4^{3-}$ , $NO_3^-$ .

n = 0 - 10

Erfindungsgemäß besonders bevorzugt sind die Anionen

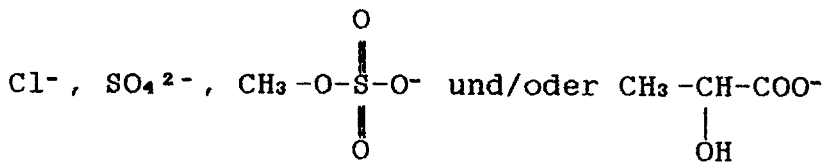

wobei auch mehrere Anionen nebeneinander vorliegen können.

Die erfindungsgemäßen Verbindungen der allgemeinen Formeln (6) und (7) können eingesetzt werden als Komponenten für die Herstellung von Polyestern, Polyesteramiden, Polyurethanen oder Polyepoxiden, welche Verwendung finden als flexible Überzüge, Formkörper, Dichtungsmassen, faserverstärkten Verbundwerkstoffen.

Weiterhin sind sie von Vorteil bei der Formulierung von Emulgatoren auf dem Gebiet der Landwirtschaft, der Metallverarbeitung und zum Teil in der Kosmetik.

Die erfindungsgemäßen Verbindungen der allgemeinen Formel (1) mit $k + l > 0$ finden insbersondere Verwendung als Antimikrobika. Sie eignen sich als Konservierungsmittel oder Desinfektionsmittel für technische Produkte aller Art im Pflanzenschutz, in der Landwirtschaft oder in der Kosmetik.

Ebenfalls konserviert werden können Leime, Anstrichsmittel oder Farben auf Basis organischer Bindemittel. Die Verwendung zum Holzschutz ist ebenfalls möglich.

Durch Kombination der Verbindungen der allgmeinen Formel (1) mit oberflächenaktiven Stoffen erhält man Wasch- und Reinigungsmittel mit antibakterieller Wirkung , mit denen Textilien antimikrobiell ausgerüstet werden können.

Die in den nachfolgenden Beispielen angewandten Analysenmethoden sind die auf diesem Gebiet allgemein üblich und sind im einzelnen:

1. Gesamtaminzahl (GAZ), Tertiäraminzahl (TAZ)

Die Gesamtaminzahl gibt die Anzahl Milligramm Kaliumhydroxid an, die der Gesamtaminbasizität von 1 g der Aminverbindung equivalent sind (mg KOH/g). Die Tertiäraminzahl gibt die Anzahl Milligramm Kaliumhydroxid an, die der Tertiäraminbasizität von 1 g der Aminverbindung equivalent sind.

Die Werte werden bestimmt nach A.O.C.S. Official Method Tf 2a - 64.

2. Verseifungszahl (VZ)

Die Verseifungszahl ist ein Maß für die in Fetten und technischen Fettsäuren enthaltenen freien und gebundenen Säuren. Sie gibt die Anzahl Milligramm Kaliumhydroxid an, die notwendig ist, um 1 Gramm Fett oder technische Fettsäuren zu verseifen. (mg KOH/g).

Die Werte werden bestimmt nach den Einheitsmethoden der Deutschen Gesellschaft für Fettchemie (DGF): DGF C-V3.

3. Hydroxylzahl (OHZ)

Die Hydroxylzahl dient zur Ermittlung des Gehalts an Hydroxylgruppen und gibt die Anzahl Milligramm Kaliumhydroxid an, die notwendig ist, um die von 1 Gramm Fett bei der Acetylisierung verbrauchte Essigsäure zu neutralisieren (mg KOH/g).

Die Werte werden bestimmt nach der DGF-Einheitsmethode C-V17a.

4. Säurezahl (SZ)

Die Säurezahl ist ein Maß für den Gehalt eines Fettes oder technischen Fettsäuren an freien Säuren und gibt die Milligramm Kaliumhydroxid an, die notwendig ist, um 1 Gramm Substanz zu neutralisieren.

Die Werte werden bestimmt nach der DGF-Einheitsmehtode C-V4.

5. Gehalt an Kationenaktiv-Substanz Cat $SO_3$

Diese Methode dient zur Bestimmung des Gehalts an kationenaktiven Substanzen. Kationenaktive Substanzen sind hier kurz- und langkettige Verbindungen, welche primäre, sekundäre, tertiäre Aminogruppen oder Ammoniumgruppen enthalten. Der Gehalt wird angegben in $SO_3$ pro 100 g der Testsubstanz.

Der Gehalt wird bestimmt durch eine Zweiphasentitration gemäß ISO-Norm 2871-1 und 2871-2 (1988 E).

Beispiele

I. Herstellung der Hydroxylamine der allgemeinen Formel (4) und (5)

Beispiel 1

912 g (2 mol) eines Amins der allgemeinen Formel (2) mit

$$a + c = 6{,}6$$
$$b = 0$$

wurde in einem Autoklaven bei 145 - 160 °C portionsweise mit 352 g (8 mol) Ethylenoxid versetzt, so daß der Druck zwischen 1 - 3 bar gehalten wurde. Nachdem die zugegebene Menge Ethylenoxid abreagiert war, erhielt man 1.264 g einer hellen Flüssigkeit der allgemeinen Formel (4) mit

$$a + c = 6{,}6$$
$$b = 0$$

$a + e + f + d = 4$ und
$R^3, R^4, R^7, R^8 = H$

Diese Verbindung hatte eine Gesamtaminzahl (GAZ) von 179 mg KOH/g, eine Tertiäraminzahl (TAZ) von 175 mg KOH/g und eine Hydroxylzahl (OHZ) von 348 mg KOH/g.

Beispiel 2

912 g (2 mol) eines Amins der allgemeinen Formel (2) mit

$$a + c = 6{,}6$$
$$b = 0$$

wurden in einem Autoklaven bei 145 - 160 °C portionsweise mit 352 g (8 mol) Ethylenoxid alkyliert, so daß der Druck bei 1 - 3 bar gehalten wurde. Nachem das zugegebene Ethylenoxid abreagiert war, wurden 0,2 g KOH zur Reaktionsmischung gegeben und weitere 352 g (8 mol) Ethylenoxid wie oben beschrieben zugegeben und abreagiert. Es entstanden 1.616 g einer hellen Flüssigkeit der allgemeinen Formel (4) mit

$$a + c = 6{,}6$$
$$b = 0$$
$$d + e + f + g = 8 \quad \text{und}$$
$$R^3, R^4, R^7, R^8 = H$$

Die Analysenzahlen dieser Verbindung waren:

GAZ = 141 mg KOH/g
TAZ = 141 mg KOH/g
OHZ = 296 mg KOH/g

Beispiel 3

237 g (1 mol) eines Amins der allgemeinen Formel (2) mit

$$a + c = 2,8$$
$$b = 0$$

wurde - wie unter Beispiel 1 angegeben - mit 176 g (4 mol) Ethylenoxid alkyliert. Es resultierten 413 g einer hellen Flüssigkeit der allgemeinen Formel (4) mit

$$a + c = 2,8$$
$$b = 0$$
$$d + e + f + g = 4 \quad \text{mit}$$
$$R^3, R^4, R^7, R^8 = H$$

Die Verbindung hatte folgende Analysenzahlen:
GAZ = 275 mg KOH/g
TAZ = 268 mg KOH/g
OHZ = 567 mg KOH/g

Beispiel 4

237 g (1 mol) eines Amins der allgemeinen Formel (2) mit

$$a + c = 2,8$$
$$b = 0$$

wurden - wie unter Beispiel 1 beschrieben - mit 352 g (8 mol) Ethylenoxid alkyliert. Es entstanden 589 g einer hellen Flüssigkeit der allgemeinen Formel (4) mit

$$a + c = 2,8$$
$$b = 0$$
$$d + e + f + g = 8 \quad \text{und}$$
$$R^3, R^4, R^7, R^8 = H$$

Diese Verbindung hatte folgende Analysenzahlen:
GAZ = 197 mg KOH/g
TAZ = 197 mg KOH/g
OHZ = 439 mg KOH/g

Beispiel 5

615 g (1 mol) des Amin der allgemeinen Formel (2) mit

$$a + c = 2,5$$
$$b = 9$$

wurde - wie unter Beispiel 1 beschrieben - mit 176 g (4 mol) Ethylenoxid alkyliert. Es entstanden 791 g einer hellen Flüssigkeit mit der allgemeinen Formel (4) mit

$$a + c = 2,5$$
$$b = 9$$
$$d + e + f + g = 4 \quad \text{und}$$
$$R^3, R^4, R^7, R^8 = H$$

Die Verbindung hatte folgende Analysenzahlen:
GAZ = 144 mg KOH/g
TAZ = 142 mg KOH/g
OHZ = 291 mg KOH/g

Beispiel 6

550 g (1 mol) eines Amins der allgemeinen Formel (3) mit

$$a = 7,7$$
$$b = 1$$
$$R^6 = -CH_3$$

wurden wie in Beispiel 1 beschrieben mit 132 g (3 mol) Ethylenoxid alkyliert. Es entanden 682 g einer hellen Flüssigkeit der Allgemeinen Formel (5) mit

$$a = 7,7$$
$$b = 1$$
$$R^6 = -CH_3$$
$$d + e = 3 \quad \text{und}$$
$$R^3, R^4 = H$$

Die Verbindung hatte folgende Analysezahlen:
GAZ = 84 mg KOH/g
TAZ = 84 mg KOH/g
OHZ = 180 mg KOH/g

Beispiel 7

912 g (2 mol) eines Amins der allgemeinen Formel (2) mit

$$a + c = 6,6$$
$$b = 0$$

wurden wie unter Beispiel 1 angegeben mit 464 g (8 mol) Propylenoxid alkoxyliert. Es resultierten 1.376 g einer hellen Flüssigkeit der allgemeinen Formel (4) mit

$$a + c = 6{,}6$$
$$b = 0$$
$$d + e + f + g = 4$$
$$R^3, R^4, R^7, R^8 = -CH_3$$

Die Verbindung hatte folgende Analysenwerte:
GAZ = 163 mg KOH/g
TAZ = 162 mg KOH/g
OHZ = 323 mg KOH/g

II Herstellung der Esteramine der allgemeinen Formeln (6) und (7)

Beispiel 8

625 g (1 mol) des Aminethoxylats aus Beispiel 1 wurden mit 570 g (2 mol) Talgmethylester ($C_{17}H_{35}COOCH_3$), 1,5 g festem, pulverförmigem NaOH und 3 g Natriumhypophosphit versetzt und unter einer Stickstoffatmosphäre gerührt und auf 180 °C aufgeheizt. Das während der Reaktion entstehende Methanol wurde abdestilliert. Nachdem ca 90 % der theoretischen Methanolmenge entfernt waren, wurde ein Vakuum von ca. 20 mbar angelegt und die Umesterung vervollständigt. Nach ca. 7 Stunden entstanden 1.135 g einer gelben Flüssigkeit der allgemeinen Formeln (6) mit

$$a + c = 6{,}6$$
$$b = 0$$
$$d + e + f + g = 4$$
$$R^3, R^4, R^7, R^8 = H$$
$$R^1, R^2, R^9, R^{10} = 2 \times H,\ 2 \times -\underset{\underset{O}{\|}}{C}-C_{17}H_{35}$$

Die Verbindung hatte folgende Analysenzahlen:
GAZ = 98 mg KOH/g
TAZ = 97 mg KOH/g
OHZ = 93 mg KOH/g
VZ = 106 mg KOH/g (VZ = Verseifungszahl)

Beispiel 9

625 g (1 mol) des Aminethoxylats aus Beispiel 1 wurden mit 855 g (3 mol) Talgmethylester, 2 g NaOH, 4 g Natriumhypophosphit - wie unter Beispiel 8 beschrieben - umgeestert. Es entstanden 1.390 g einer gelben Flüssigkeit nach der allgemeinen Formel (6) mit

$$a + c = 6,6$$
$$b = 0$$
$$d + e + f + g = 4$$
$$R^3, R^4, R^7, R^8 = H$$
$$R^1, R^2, R^9, R^{10} = 1 \times H,\ 3 \times -\underset{\underset{O}{\|}}{C}-C_{17}H_{35}$$

Die Analysenzahlen dieser Verbindung sind:

GAZ = 78 mg KOH/g
TAZ = 74 mg KOH/g
OHZ = 57 mg KOH/g
VZ = 117 mg KOH/g

Beispiel 10

799 g (1 mol) des Aminethoxylats aus Beispiel 2 wurden mit 570 g (2 mol) Talgmethylester, 2 g NaOH und 4 g Natiumhypophosphit - wie unter Beispiel 8 beschrieben - umgeestert. Es entstanden 1.311 g einer gelben Flüssigkeit der allgemeinen Formel (6) mit

$$a + c = 6,6$$
$$b = 0$$
$$d + e + f + g = 8$$
$$R^3, R^4, R^7, R^8 = H$$
$$R^1, R^2, R^9, R^{10} = 2 \times H,\ 2 \times -\underset{\underset{O}{\|}}{C}-C_{17}H_{35}$$

Die Analysezahlen dieser Verbindung sind:

GAZ = 89 mg KOH/g
TAZ = 88 mg KOH/g
OHZ = 88 mg KOH/g
VZ = 82 mg KOH/g

Beispiel 11

775 g (1 mol) des Aminethoxylats nach Beispiel 5 wurden mit 570 g (2 mol) Talgmethylester, 1,5 g NaOH und 3 g Natriumhypophosphit - wie unter Beispiel 8 beschrieben - umgeestert. Es entstanden 1.285 g einer gelben Flüssigkeit der allgemeinen Formel (6) mit

$a + c = 2,5$

$b = 9$

$d + e + f + g = 4$

$R^3, R^4, R^7, R^8 = H$

$R^1, R^2, R^9, R^{10} = 2 \times H, 2 \times -\underset{\overset{\|}{O}}{C}-C_{17}H_{35}$

Die Verbindung hatte folgende Analysenzahlen:

GAZ = 86 mg KOH/g
TAZ = 85 mg KOH/g
OHZ = 86 mg KOH/g
VZ = 91 mg KOH/g

Beispiel 12

407 g (1 mol) des Aminethoxylats nach Beispiel 3 wurden mit 570 g (2 mol) Talgmethylester, 1,1 g NaOH und 2,3 g Natriumhypophosphit - wie unter Beispiel 8 beschrieben - umgeestert. Es entstanden 916 g einer gelben Flüssigkeit der allgemeinen Formel (6) mit

$a + c = 2,8$

$b = 0$

$d + e + f + g = 4$

$R^3, R^4, R^7, R^8 = H$

$R^1, R^2, R^9, R^{10} = 2 \times H, 2 \times -\underset{\overset{\|}{O}}{C}-C_{17}H_{35}$

Die Verbindung hatte folgende Analysenzahlen:

GAZ = 121 mg KOH/g
TAZ = 121 mg KOH/g
OHZ = 118 mg KOH/g
VZ = 127 mg KOH/g

Beispiel 13

625 g (1 mol) des Aminethoxylats nach Beispiel 1 wurden mit 539 g (2 mol) Talgfettsäure und 1,2 g Hypophosphorige Säure unter einer Stickstoffatmosphäre gerührt und auf 180 °C aufgeheizt. Das während der Reaktion entstandene Wasser wurde abdestilliert. Nachdem eine Säurezahl von ca. 10 erreicht war, wurde ein Vakuum von ca. 20 mbar angelegt und die Reaktion weitergeführt bis die Säurezahl < 2 war. Es entstanden 1.129 g einer hellen Flüssigkeit der allgemeinen

Formel (6) mit

$$a + c = 6{,}6$$
$$b = 0$$

$$d + e + f + g = 4$$
$$R^3, R^4, R^7, R^8 = H$$
$$R^1, R^2, R^9, R^{10} = 2 \times H,\ 2 \times -\underset{\overset{\|}{O}}{C}-C_{17}H_{35}$$

Die Verbindung hatte folgende Analysenzahlen:

GAZ = 93 mg KOH/g
TAZ = 93 mg KOH/g
OHZ = 95 mg KOH/g
VZ = 98 mg KOH/g
SZ = 1,3 mg KOH/g (SZ = Säurezahl)

Beispiel 14

1.669 g (2,5 mol) des Aminoalkohols nach Beispiel 6 wurden mit 1.069 g (3.75 mol) Talgmethylester, 2,8 g NaOH und 5,8 g Natriumhypophosphit - wie unter Beispiel 8 beschrieben - umgeestert. Es entstanden 2.626 g einer gelben klaren Flüssigkeit der allgemeinen Formel (7) mit

$$a = 7{,}7$$
$$b = 1$$
$$R^6 = -CH_3$$

$$d + e = 3$$
$$R^3, R^4 = H$$
$$R^1, R^2 = 0{,}5 \times H,\ 1{,}5 \times -\underset{\overset{\|}{O}}{C}-C_{17}H_{35}$$

Die Verbindung hatte folgende Analysenzahlen:

GAZ = 55 mg KOH/g
TAZ = 55 mg KOH/g
OHZ = 41 mg KOH/g
VZ = 76 mg KOH/g

Beispiel 15

625 g (1 mol) des Aminethoxylats aus Beispiel 1 wurden mit 1.140 g (4 mol) Talgmethylester, 2 g NaOH, 3 g Natriumhypophosphit wie unter Beispiel 8 beschrieben umgesetzt. Es entstanden 1.642 g einer gleben Flüssigkeit der allgemeinen Formel (6) mit

$$a + c = 6,6$$
$$b = 0$$
$$d + e + f + g = 4$$
$$R^3, R^4, R^7, R^8 = H$$
$$R^1, R^2, R^9, R^{10} = 4 \times -\underset{\underset{O}{\|}}{C}-C_{17}H_{35}$$

Das Reaktionsprodukt hatte folgende Analysenwerte:
GAZ = 68 mg KOH/g
TAZ = 68 mg KOH/g
OHZ = 1,5 mg KOH/g
VZ = 139 mg KOH/g

Beispiel 16

625 g (1 Mol) des Aminethoxilats aus Beispiel 1 wurden mit 285 g (1 Mol) Talgmethylester, 1,5 g NaOH, 3,0 g Natriumhypophosphit wie unter Beispiel 8 beschrieben umgesetzt.

Es entstanden 882 g einer gelben Flüssigkeit der allgemeinen Formel (6) mit

$$a + c = 2 - 6$$
$$b = 0$$
$$d + e + f + g = 4$$
$$R^3, R^4, R^7, R^8 = H$$
$$R^1, R^2, R^9, R^{10} = 3 \times H;\ 1 \times -\underset{\underset{O}{\|}}{C}-C_{17}H_{35}$$

Das Reaktionsprodukt hatte folgende Analysenwerte:
GAZ = 128 mg KOH/g
TAZ = 128 mg KOH/g
OHZ = 64 mg KOH/g
VZ = 191 mg KOH/g

III Herstellung der quaternären Ammoniumverbindungen bzw. der Aminsalze

Beispiel 17

1.149 g (1 mol) des Esters aus Beispiel 8 wurden bei 60 °C unter Rühren mit 252 g (2 mol) Dimethylsulfat portionsweise versetzt, so daß die Temperatur der Reaktionsmischung zwischen 60 - 70 °C gehalten werden konnte. Es entstanden 1.397 g einer gelben Flüssigkeit der allgemeinen Formel (1) mit

$$a + c = 6,6$$
$$b = 0$$
$$d + e + f + g = 4$$
$$R^3, R^4, R^7, R^8 = H$$
$$R^1, R^2, R^9, R^{10} = 2 \times H,\ 2 \times -\underset{\underset{O}{\|}}{C}-C_{17}H_{35}$$
$$R^{13} = -CH_3$$
$$y^- = 2 \times {}^-\!-O\overset{\overset{O}{\|}}{\underset{\underset{O}{\|}}{S}}-OCH_3$$

Die Analysezahlen dieser Verbindung sind:

| | |
|---|---|
| Cat $SO_3$ sauer : | 9,6 g $SO_3$ / 100 g |
| GAZ : | 2,3 mg KOH/g |

Beispiel 18

1.149 g (1 mol) des Esters aus Beispiel 8 wurden bei 60 °C unter Rühren mit 200 g (2 mol) einer 90 %igen wässrigen Milchsäurelösung neutralisiert. Es entstanden 1.349 g einer gelben klaren Flüssigkeit der allgemeinen Formel (1) mit

$$a + c = 6,6$$
$$b = 0$$
$$d + e + f + g = 4$$
$$R^3, R^4, R^7, R^8 = H$$
$$R^1, R^2, R^9, R^{10} = 2 \times H,\ 2 \times -\underset{\underset{O}{\|}}{C}-C_{17}H_{35}$$
$$R^{12}, R^{13} = H$$
$$y^- = 2 \times CH_3 - \underset{\underset{OH}{|}}{CH} - COO^-$$

Die Analysezahlen dieser Verbindung sind:

| | |
|---|---|
| Cat $SO_3$ sauer : | 10,0 g $SO_3$ / 100 g |
| pH (5 %ig Isopropane/Wasser 1 : 1) : | 5,2 |

Beispiel 19

1.149 g (1 mol) des Esters aus Beispiel 8 wurden bei 60 °C unter Rühren mit 126 g (1 mol) Dimethylsulfat portionsweise versetzt, so daß die Temperatur der Reaktionsmischung zwischen 60 - 70 °C gehalten werden konnte. Anschließend gab man 100 g (1 mol) einer 90 %igen wässrigen Milchsäurelösung zur Neutralisation der Mischung hinzu. Es entstanden 1.373 g einer gelben klaren Flüssigkeit der allgemei-

nen Formel (1) mit

$$a + c = 6,6$$
$$b = 0$$
$$d + e + f + g = 4$$
$$R^3, R^4, R^7, R^8 = H$$
$$R^1, R^2, R^9, R^{10} = 2 \times H,\ 2 \times -\underset{\underset{O}{\|}}{C}-C_{17}H_{35}$$
$$R^{12}, R^{13} = 1 \times H,\ 1 \times CH_3$$

$$y^- = 1 \times {}^{-}-O\overset{\overset{O}{\|}}{\underset{\underset{O}{\|}}{S}}-OCH_3,\quad 1 \times CH_3-\underset{\underset{OH}{|}}{CH}-COO^-$$

Die Analysezahlen dieser Verbindung sind:
Cat $SO_3$ sauer : 10,1 g $SO_3$ / 100 g

Beispiel 20

1.305 g (1 mol) des Esters aus Beispiel 11 wurden - wie unter Beispiel 17 beschrieben - mit 252 g (2 mol) Dimethylsulfat quaterniert. Es entstanden 1.557 g einer gelben klaren Flüssigkeit der allgemeinen Formel (1) mit

$$a + c = 2,5$$
$$b = 9$$

$$d + e + f + g = 4$$
$$R^3, R^4, R^7, R^8 = H$$
$$R^1, R^2, R^9, R^{10} = 2 \times H,\ 2 \times -\underset{\underset{O}{\|}}{C}-C_{17}H_{35}$$
$$R^{12}, R^{13} = -CH_3$$

$$y^- = 2 \times {}^{-}-O\overset{\overset{O}{\|}}{\underset{\underset{O}{\|}}{S}}-OCH_3$$

Die Analysezahlen dieser Verbindung sind:
Cat $SO_3$ sauer : 9,0 g $SO_3$ / 100 g
GAZ : 2,2 mg KOH/g

Beispiel 21

679 g (0,65 mol) des Esters aus Beispiel 14 wurden mit 41 g (0,325 mol) Dimethylsulfat - wie im Beispiel 19 beschrieben - quaterniert und anschließend die Reaktionsmischung mit 37 g 90 %iger Milchsäure neutralisiert. Es entstanden 757 g einer gelben klaren Flüssigkeit der allgemeinen Formel (1) mit

$$a + c = 7,7$$
$$b = 1$$
$$R^6 = -CH_3$$
$$d + e = 3$$
$$R^3, R^4 = H$$
$$R^1, R^2 = 0,5 \times H,\ 1,5 \times -\underset{\underset{O}{\|}}{C}-C_{17}H_{35}$$
$$R^{12} = 0,5 \times CH_3,\ 0,5 \times -H$$

$$y^- = 0,5 \times {}^{-}-O\overset{\overset{O}{\|}}{\underset{\underset{O}{\|}}{S}}-OCH_3,\ 0,5 \times CH_3-\underset{\underset{OH}{|}}{CH}-COO^-$$

Die Analysezahlen dieser Verbindung sind:
Cat $SO_3$ sauer : 6,4 g $SO_3$ / 100 g

Beispiel 22

1.149 g (1 mol) des Esters aus Beispiel 8 wurden bei 60 °C unter gutem Rühren portionsweise mit 196 g (2 mol) einer 36 %igen wässrigen HCl-Lösung versetzt.
Es entstanden 1.345 g einer hellgelben Flüssigkeit der allgemeinen Formel (1) mit

$$a + c = 6,6$$
$$b = 0$$

$$d + e + f + g = 4$$
$$R^3, R^4, R^7, R^8 = H$$
$$R^1, R^2, R^9, R^{10} = 2 \times H,\ 2 \times -\underset{\underset{O}{\|}}{C}-C_{17}H_{35}$$
$$R^{12}, R^{13} = H$$
$$y^- = 2 \times Cl^-$$

Die Analysenzahlen dieser Verbindungen sind:

| | |
|---|---|
| Cat $SO_3$ sauer : | 10,3 g $SO_3$ / 100 g |
| pH (5 %ig Isopropanol / Wasser 1 : 1) : | 4,2 |

**Patentansprüche**

1. Verbindungen der allgemeinen Formel

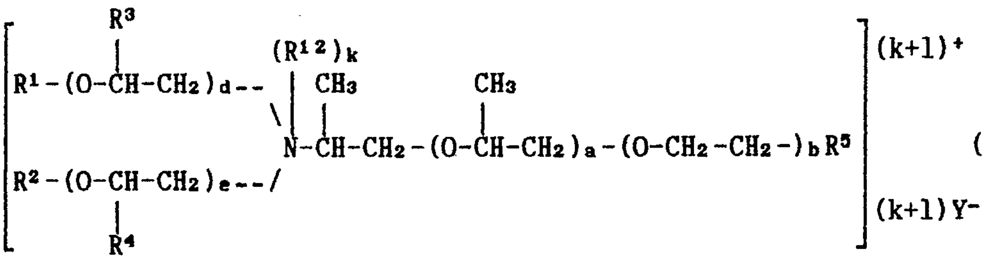

( 1 )

in welcher $R^5$ ein Rest

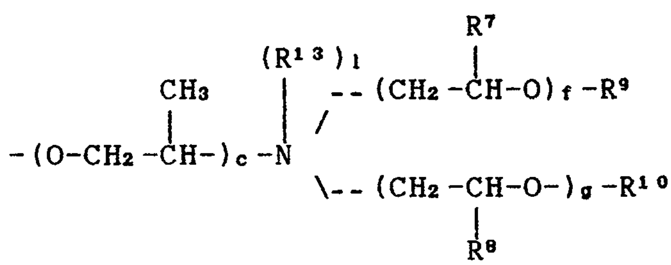

oder -OH oder -O-$R^6$ sein kann, mit $R^6$ = Alkylrest mit 1-5 C-Atomen oder ein Rest

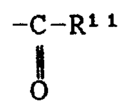

und worin $R^1$, $R^2$, $R^9$, $R^{10}$ unabhängig voneinander Wasserstoff, ein Alkylrest mit 1-5 Kohlenstoffatomen oder der Rest

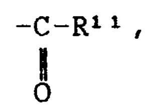

in welchem $R^{11}$ ein gegebenenfalls verzweigter, gegebenenfalls Mehrfachbindungen oder Hydroxylgruppen enthaltender Kohlenwasserstoffrest mit 11 - 21 Kohlenstoffatomen ist, mit der Bedingung, daß mindestens einer der Reste $R^1$, $R^2$, $R^9$, $R^{10}$ der Rest

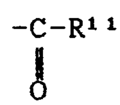

sein muß und $R^3$, $R^4$, $R^7$, $R^8$ unabhängig voneinander -H oder $CH_3$ sein können und $R^{12}$, $R^{13}$ unabhängig voneinander -H, -$CH_3$, -$C_2H_5$ oder ein Benzylrest sein können und k und l = 0 bis 1 und k + l = 0 bis 2 und a + c = 1 bis 40, b = 0 bis 40, d + e + f + g = 2 bis 12 sind und $y^-$ für ein ein- oder mehrwertiges Anion steht mit insgesamt k + l negativen Ladungen.

2. Verbindungen gemäß Anspruch 1, dadurch gekennzeichnet, daß k + l = 0 ist.

3. Verbindungen gemäß Anspruch 1, dadurch gekennzeichnet, daß $R^{12}$, $R^{13}$ gleich oder verschieden H oder $-CH_3$ sind und k + l = 0,5 bis 2 ist.

4. Verbindungen gemäß Ansprüche 1 bis 4, dadurch gekennzeichnet, daß $R^5$ der Rest

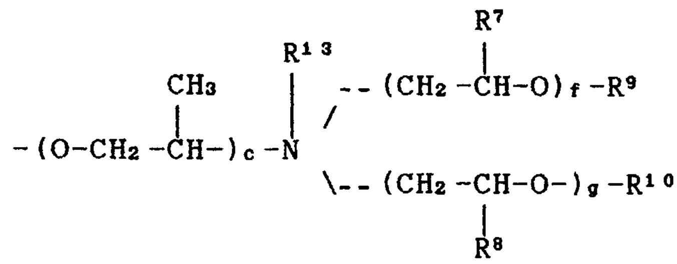

ist.

5. Verbindungen gemäß Ansprüche 1 bis 3, dadurch gekennzeichnet, daß $R^5$ der Rest $-O-R^6$ ist, worin $R^6$ ein Alkylrest mit 1 bis 5 C-Atomen sein kann.

6. Verbindungen gemäß Ansprüche 1 bis 5, dadurch gekennzeichnet, daß zumindest einer der Reste $R^1$, $R^2$, $R^9$, $R^{10}$ der Rest

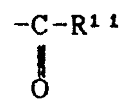

ist, worin $R^{11}$ ein Alkylrest oder Alkylenrest mit 7 bis 17 Kohlenstoffatomen sein kann.

7. Verbindungen gemäß Ansrüche 1 bis 6, dadurch gekennzeichnet, daß $R^{12}$, $R^{13}$ = $-CH_3$ und/oder H sind mit k + l = 0,5 bis 2, d + e + f + g = 4 bis 8 sind, $R^3$, $R^4$, $R^7$, $R^8$ = H bedeuten und 1 bis 3 der Reste $R^1$, $R^2$, $R^9$, $R^{10}$ die Bedeutung

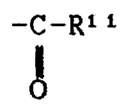

haben.

8. Verbindungen gemäß Ansprüche 1 bis 7, dadurch gekennzeichnet, daß a + c = 1 bis 15 und b = 0 bis 15 ist.

9. Verfahren zur Herstellung der Verbindungen gemäß Anspruch 1, dadurch gekennzeichnet, daß in erster Stufe Verbindungen der allgemeinen Formel (2) und/oder (3)

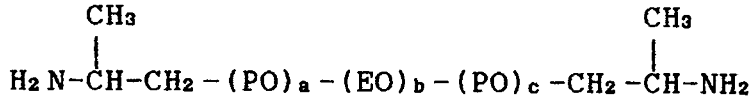

( 2 )

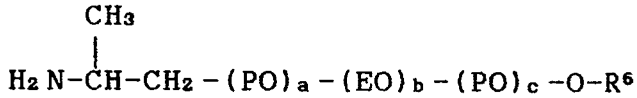

( 3 )

worin

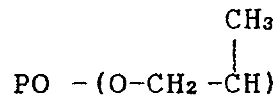

und EO -(O-$CH_2$-$CH_2$)- bedeuten und worin a = 0 bis 20, c = 0 bis 20 und a + c, b und $R^6$ die oben angegebene Bedeutung haben, gegebenenfalls unter Verwendung von Lösungsmitteln mit einer dem gewünschten Alkoxilierungsgrad entsprechenden Menge an Alkylenoxid bei 120 bis 160 °C gegebenenfalls in Gegenwart von Katalysatoren in einem Druckreaktor bei 1 - 4 bar mit einer dem gewünschten Akoxilierungsgrad entsprechenden Menge an Alkylenoxid abreagiert und anschließend die gebildeten Verbindungen der allgemeinen Formeln (4) und/oder (5)

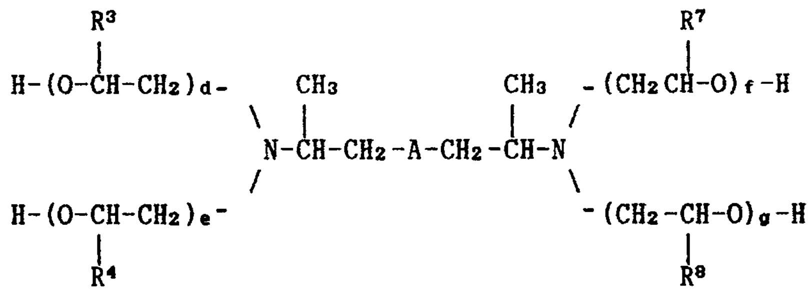

( 4 )

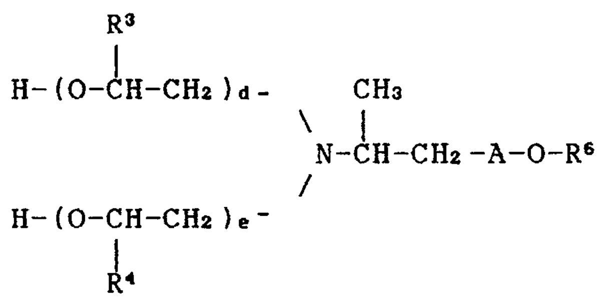

( 5 )

worin A -$(PO)_a$-$(EO)_b$-$(PO)_c$- bedeutet, mit einer dem gewünschten Veresterungsgrad entsprechenden Menge an Fettsäure oder Fettsäurederivat, gegebenenfalls in Gegenwart eines Katalysators bei 160 - 200 °C reagieren läßt und das sich bildende Reaktionswasser, bzw. den Alkolhol, kontinuierlich, gegebenenfalls bei einem Druck < 1 bar abdestilliert und gegebenenfalls anschließend die gebildeten Verbindungen der allgemeinen Formeln (6) und/oder (7)

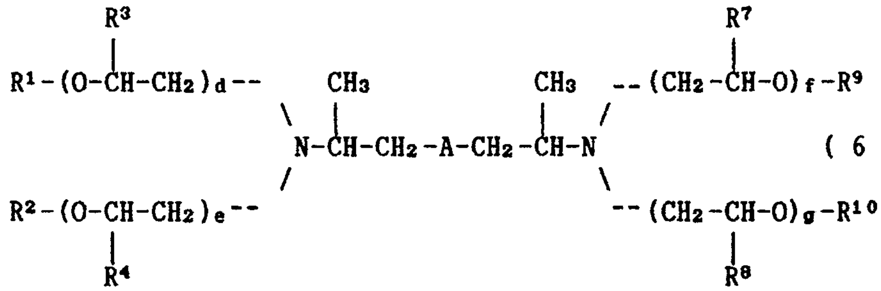

( 6 )

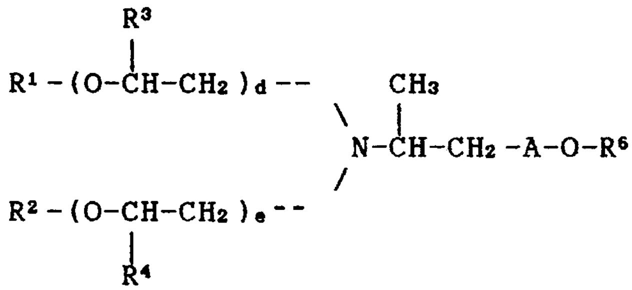

( 7 )

portionsweise mit einem Quaternierungsmittel und/oder mit einer Säure, gegebenenfalls unter Mitverwendung von Lösungsmitteln, bei 20 - 80 °C in einer dem gewünschten Quaternierungs-(Salz-)grad entsprechenden Menge zu den Verbindungen der allgemeinen Formel (1) mit k + l > 0 umsetzt.

**10.** Verwendung der Verbindungen gemäß Ansprüche 1 bis 9 als Komponenten für die Herstellung von Emulgatoren, Reinigungsmitteln, Desinfektionsmitteln, Konservierungsmitteln.

**Patentansprüche für folgende Vertragsstaat: ES**

**1.** Verfahren zur Herstellung von Verbindungen der allgemeinen Formel

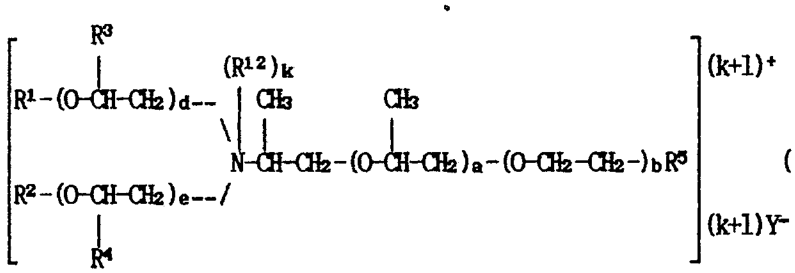

( 1 )

in welcher $R^5$ ein Rest

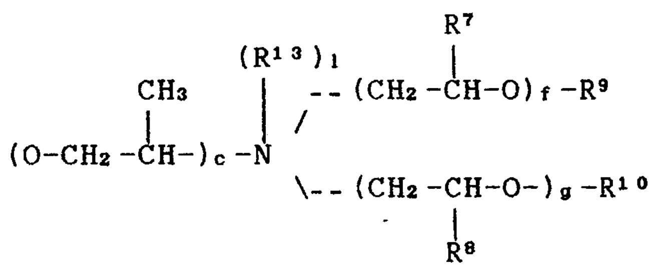

oder -OH oder -O-$R^6$ sein kann, mit $R^6$ = Alkylrest mit 1-5 C-Atomen oder ein Rest

$$-\underset{\underset{O}{\|}}{C}-R^{11}$$

und worin $R^1$, $R^2$, $R^9$, $R^{10}$ unabhängig voneinander Wasserstoff, ein Alkylrest mit 1-5 Kohlenstoffatomen oder der Rest

$$-\underset{\underset{O}{\|}}{C}-R^{11},$$

in welchem $R^{11}$ ein gegebenenfalls verzweigter, gegebenenfalls Mehrfachbindungen oder Hydroxylgruppen enthaltender Kohlenwasserstoffrest mit 11 - 21 Kohlenstoffatomen ist, mit der Bedingung, daß mindestens einer der Reste $R^1$, $R^2$, $R^9$, $R^{10}$ der Rest

$$-\underset{\underset{O}{\|}}{C}-R^{11}$$

sein muß und $R^3$, $R^4$, $R^7$, $R^8$ unabhängig voneinander -H oder $CH_3$ sein können und $R^{12}$, $R^{13}$ unabhängig voneinander -H, -$CH_3$, -$C_2H_5$ oder ein Benzylrest sein können und k und l = 0 bis 1 und k + l = 0 bis 2 und a + c = 1 bis 40, b = 0 bis 40, d + e + f + g = 2 bis 12 sind und $y^-$ für ein ein- oder mehrwertiges Anion steht mit insgesamt k + l negativen Ladungen, wobei in erster Stufe Verbindungen der allgemeinen Formel (2) und/oder (3)

$$H_2N-\overset{CH_3}{\overset{|}{C}}H-CH_2-(PO)_a-(EO)_b-(PO)_c-CH_2-\overset{CH_3}{\overset{|}{C}}H-NH_2 \qquad (2)$$

$$H_2N-\overset{CH_3}{\overset{|}{C}}H-CH_2-(PO)_a-(EO)_b-(PO)_c-O-R^6 \qquad (3)$$

worin

$$PO \quad -(O-CH_2-\overset{CH_3}{\overset{|}{C}}H)$$

und EO -(O-$CH_2$-$CH_2$)- bedeuten und worin a = 0 bis 20, c = 0 bis 20 und a + c, b und $R^6$ die oben angegebene Bedeutung haben, gegebenenfalls unter Verwendung von Lösungsmitteln mit einer dem gewünschten Alkoxilierungsgrad entsprechenden Menge an Alkylenoxid bei 120 bis 160 °C gegebenenfalls in Gegenwart von Katalysatoren in einem Druckreaktor bei 1 - 4 bar mit einer dem gewünschten Akoxilierungsgrad entsprechenden Menge an Alkylenoxid abreagiert und anschließend die gebildeten Verbindungen der allgemeinen Formeln (4) und/oder (5)

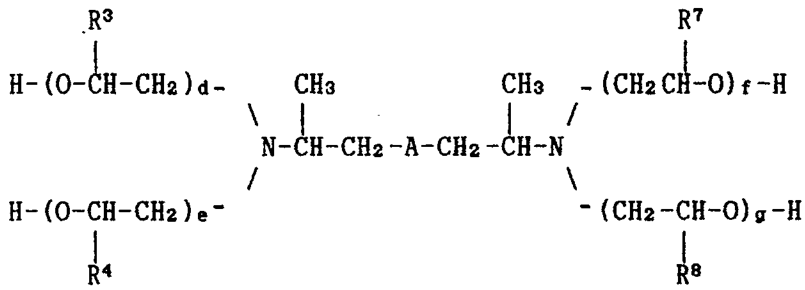

( 4 )

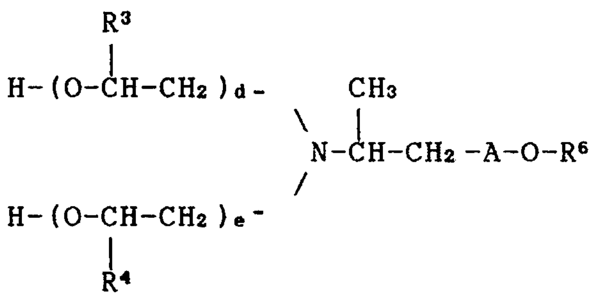

( 5 )

worin A $-(PO)_a-(EO)_b-(PO)_c-$ bedeutet, mit einer dem gewünschten Veresterungsgrad entsprechenden Menge an Fettsäure oder Fettsäurederivat, gegebenenfalls in Gegenwart eines Katalysators bei 160 - 200 °C reagieren läßt und das sich bildende Reaktionswasser, bzw. den Alkolhol, kontinuierlich, gegebenenfalls bei einem Druck < 1 bar abdestilliert und gegebenenfalls anschließend die gebildeten Verbindungen der allgemeinen Formeln (6) und/oder (7)

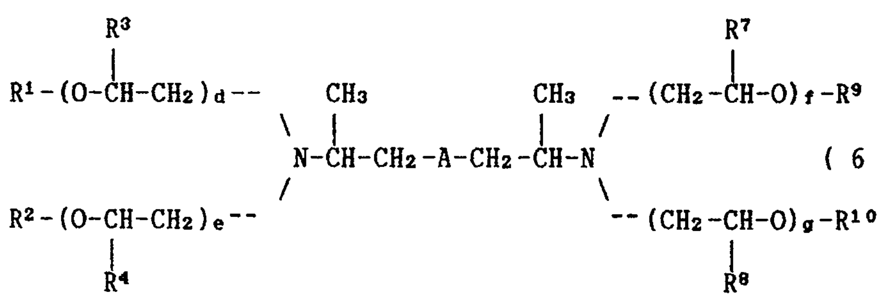

( 6 )

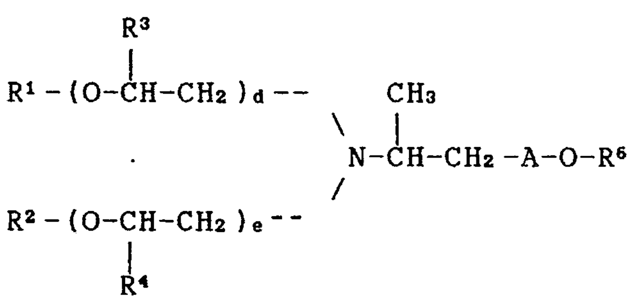

( 7 )

portionsweise mit einem Quaternierungsmittel und/oder mit einer Säure, gegebenenfalls unter Mitverwendung von Lösungsmitteln, bei 20 - 80 °C in einer dem gewünschten Quaternierungs-(Salz-)grad entsprechenden Menge zu den Verbindungen der allgemeinen Formel (1) mit k + l > 0 umsetzt.

2. Verfahren zur Herstellung von Verbindungen der allgemeinen Formel 1, dadurch gekennzeichnet, daß $k + l = 0$ ist.

3. Verfahren zur Herstellung von Verbindungen der allgemeinen Formel 1, dadurch gekennzeichnet, daß $R^{12}$, $R^{13}$ gleich oder verschieden H oder $-CH_3$ sind und $k + l = 0{,}5$ bis 2 ist.

4. Verfahren zur Herstellung von Verbindungen der allgemeinen Formel 1, dadurch gekennzeichnet, daß $R^5$ der Rest

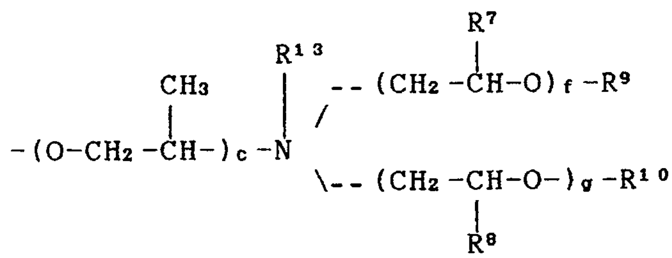

ist.

5. Verfahren zur Herstellung von Verbindungen der allgemeinen Formel 1, dadurch gekennzeichnet, daß $R^5$ der Rest $-O-R^6$ ist, worin $R^6$ ein Alkylrest mit 1 bis 5 C-Atomen sein kann.

6. Verfahren zur Herstellung von Verbindungen der allgemeinen Formel 1, dadurch gekennzeichnet, daß zumindest einer der Reste $R^1$, $R^2$, $R^9$, $R^{10}$ der Rest

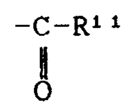

ist, worin $R^{11}$ ein Alkylrest oder Alkylenrest mit 7 bis 17 Kohlenstoffatomen sein kann.

7. Verfahren zur Herstellung von Verbindungen der allgemeinen Formel 1, dadurch gekennzeichnet, daß $R^{12}$, $R^{13}$ = $-CH_3$ und/oder H sind mit $k + l = 0{,}5$ bis 2, $d + e + f + g = 4$ bis 8 sind, $R^3$, $R^4$, $R^7$, $R^8$ = H bedeuten und 1 bis 3 der Reste $R^1$ $R^2$, $R^9$, $R^{10}$ die Bedeutung

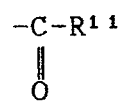

haben.

8. Verfahren zur Herstellung von Verbindungen der allgemeinen Formel 1, dadurch gekennzeichnet, daß $a + c = 1$ bis 15 und $b = 0$ bis 15 ist.

9. Verwendung der Verbindungen der gemäß Ansprüche 1 bis 9 hergestellten Verbindungen als Komponenten für die Herstellung von Emulgatoren, Reinigungsmitteln, Desinfektionsmitteln, Konservierungsmitteln.

Europäisches Patentamt

# EUROPÄISCHER RECHERCHENBERICHT

Nummer der Anmeldung

EP 91 10 8091

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | US-A-2 831 813 (MATUSZAK et al.)<br>* Spalte 2, Zeilen 20-45 *<br>– – – | 1,2,6,8,9 | C 07 C 219/06<br>C 11 D 1/46<br>A 01 N 37/12 |
| A | US-A-3 398 163 (MEYERS et al.)<br>* Das ganze Dokument *<br>– – – | 1,2,6,<br>8-10 | |
| A | US-A-2 970 158 (LEVIS)<br>* Das ganze Dokument *<br>– – – | 1-10 | |
| A | US-A-2 173 058 (KRITCHEVSKY)<br>* Das ganze Dokument *<br>– – – | 1-10 | |
| A | EP-A-0 131 865 (HOECHST)<br>* Beispiele; Ansprüche *<br>– – – | 1-9 | |
| A,P | WO-A-9 101 295 (HENKEL)<br>* Beispiele; Ansprüche *<br>– – – – – | 1-10 | |

| RECHERCHIERTE SACHGEBIETE (Int. Cl.5) |
|---|
| C 07 C 219/00 |

Der vorliegende Recherchenbericht wurde für alle Patentansprüche erstellt

| Recherchenort | Abschlußdatum der Recherche | Prüfer |
|---|---|---|
| Den Haag | 11 September 91 | HELPS I.M. |

KATEGORIE DER GENANNTEN DOKUMENTE

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze

E: älteres Patentdokument, das jedoch erst am oder nach dem Anmeldedatum veröffentlicht worden ist
D: in der Anmeldung angeführtes Dokument
L: aus anderen Gründen angeführtes Dokument

&: Mitglied der gleichen Patentfamilie, übereinstimmendes Dokument